# EUROPEAN PATENT SPECIFICATION

(11) **EP 1 377 249 B1**
(45) Date of publication and mention of the grant of the patent: **27.06.2007**
(21) Application number: 02705636.5
(22) Date of filing: 19.03.2002
(51) Int. Cl.: A61H 39/00, A61F 7/00

(54) **DEVICE FOR ACUPUNCTURIC TREATMENT**
VORRICHTUNG FÜR EINE AKUPUNKTURBEHANDLUNG
DISPOSITIF POUR TRAITEMENT D'ACUPUNCTURE

(30) Priority: 21.03.2001 NO 20011427
(43) Date of publication of application: 07.01.2004
(73) Proprietor: Bergersen, Turid, 0478 Oslo (NO)
(72) Inventor: Bergersen, Turid, 0478 Oslo (NO)
(74) Representative: Jostarndt, Hans-Dieter
(86) International application number: PCT/NO2002/000114
(87) International publication number: WO 2002/074225

(56) References cited:
- DE-A1- 4 129 229
- GB-A- 2 216 800
- GB-A- 2 244 432
- US-A- 4 920 956

## Description

The present invention regards a device for acupressuric treatment, more specifically for stimulating the acupoints on both sides of the root of the nose and over the eyes.

So-called acupoints, that is acupuncture points and acupressure points, are known to exist around the eyes and on both sides of the nose, and it is also known that pressure on these points may alleviate head aches, pressure in the forehead, tiredness and irritation of the eyes, as well as other afflictions.

Glasses are known from the patent literature, which stimulate acupoints around or near the eyes in order to improve blood circulation and cell activity, thereby to alleviate afflictions such as colds, bronchitis, headaches and concentration problems. Furthermore, devices for acupressuric treatment are known from the patent literature, which devices stimulate acupoints on both sides of the nose and on the bridge of the nose in order to alleviate e.g. breathing problems (blocked nose).

From US-A1 4 920 956 there is known a mask-like acupressure device, the purpose of which is to alleviate afflictions such as infections of the nose. In accordance with a first embodiment, the device consists of a liquid-filled mask that may be heated and then placed on the nose. In accordance with a second embodiment, the device consists of a spectacle-like device with three electrodes that create and transmit heat to the desired acupressure points. Those points stimulated by this known device are different from those intended to be stimulated by device in accordance with the invention.

GB 2 216 800 A shows a mask-like device for massaging the muscles around the eyes in order to improve the blood circulation in these areas. The device consists of a flexible belt having a series of electrically connected elements, which elements are arranged in positions corresponding to a number of known acupuncture points around the eyes. These elements are connected to a low frequency generator, such that the elements will press against and massage the relevant acupuncture points.

GB 2 224 432 A shows a similar device to that of GB 2 216 800 A, and describes the principle of operation of the massaging elements in greater detail.

DE 4 129 229 A1 describes a device for therapeutic treatment using electroacupuncture.

CN-A 87 101 345 describes stimulation of several named acupuncture points for treatment of myopia.

The present invention regards a device of the type mentioned by way of introduction, the purpose of which is to offer a better effect than that achieved by the previously known devices. For this purpose, the invention regards a device for acupuncturic treatment, more specifically for stimulating acupoints on both sides of the root of the nose and over the eyes near the root of the nose, that is the acupoints BL1 (Jing Ming) and BL2 (Zan Zhu), in order to alleviate the beginnings of a head ache, a migraine or sinusitis. What distinguishes the device is that a resilient, bow-shaped clip has pads arranged in pairs symmetrically about a plane of symmetry for this clip, which pads are designed to exert pressure against the desired acupoints. The pads have, in an appropriate manner, been coated or impregnated with a substance that has a cooling effect on the skin, or the pads may alternatively consist essentially of a hydrophilic gel material. One set of pads faces to one side of the resilient device in a suitable manner.

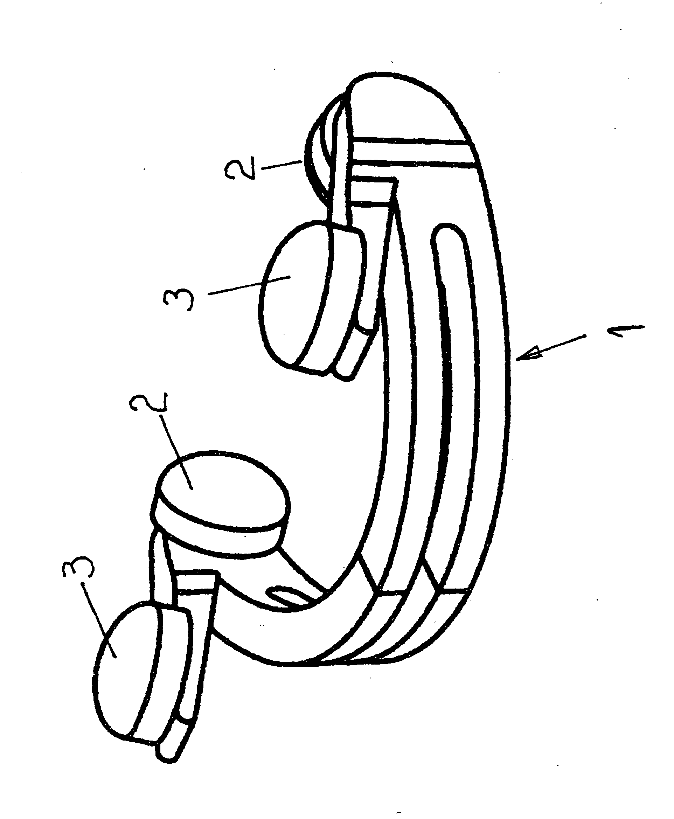

The invention will be described in greater detail with reference to the appended drawing, which is a perspective view of an example of an embodiment for an acupressure bow in accordance with the invention.

The drawing shows an example of the device in accordance with the invention for acupressuric treatment, more specifically for stimulating acupoints on both sides of the root of the nose and over the eyes, that is the acupoints BL1 (Jing Ming) and BL2 (Zan Zhu), in order to relieve the beginnings of a headache, a migraine or sinusitis. The acupoints BL1 or Jing Ming are located on either side of the root of the nose, at the innermost corner of the eye, and stimulation of these points counteracts tiredness of the eyes, e.g. "computer eyes". The acupoints BL2 or Zan Zhu are located at the innermost edge of each eyebrow in towards the nose, and stimulation of these points is effective against sinusitis and headaches located in the forehead.

The device in accordance with the invention is in the form of a bow-shaped clip 1 having pads 2 and 3 arranged in pairs symmetrically about a plane of symmetry for the bow across the plane of the bow, which pads are designed to exert pressure against said acupoints when the bow is placed over the root of the user's nose. The pads 2 act on the acupoints BL1, while the pads 3 act on the acupoints BL2. The pads 2 face each other, in towards the plane of symmetry for the clip, while the pads 3 face sideways out from the plane of the bow.

The bow 1 is constructed from a flexible compliant or resilient material, so as to give the required pressure from the pads 2 and 3 against said acupoints.

The pads 2 and 3 are in accordance with the invention constructed wholly or substantially from a workable gel material of a type that is known *per se,* whereby the pads will mould themselves to the surface against which they rest, i.e. to the skin surface at the relevant acupoints, and give a good pressure effect against the acupoint. Thus the consistency of the pads is soft and workable, although the properties change somewhat according to the ambient temperatures, but still such that the pads will have these properties at most of the temperatures at which the pads will be used.

## Claims

1. A device for acupressuric treatment, more specifically for stimulating the acupoints BL1 and BL2 on both sides of the root of the nose and over the eyes near the root of the nose, **characterised in that** a resilient, bow-shaped clip (1) has pads (2, 3) arranged in pairs symmetrically about a plane of symmetry for this clip, which pads are designed to exert pressure against said acupoints when the bow is arranged over the root of the user's nose.

2. A device in accordance with Claim 1, **characterised in that** the pads (2, 3) are coated or impregnated with a substance that has a cooling effect on the skin.

3. A device in accordance with Claim 1, **characterised in that** the pads (2, 3) consist substantially of a hydrophilic gel material.

4. A device in accordance with Claims 1- 3, **characterised in that** one set of pads (3) faces sideways to one side of the resilient device.

## Patentansprüche

1. Vorrichtung zur Akupressurbehandlung, im speziellen zur Stimulierung der Akupunkturpunkte BL1 und BL2 an beiden Seiten der Nasenwurzel und über den Augen nahe der Nasenwurzel, **dadurch gekennzeichnet, dass** eine elastische bogenförmige Klammer (1) Polster (2, 3) aufweist, die in Paaren symmetrisch zu einer Symmetrieebene für diese Klammer angeordnet sind, jene Polster sind konstruiert Druck gegen die Akupunkturpunkte auszuüben, wenn die Klammer über der Nasenwurzel des Nutzers angeordnet wird.

2. Vorrichtung gemäß Anspruch 1, **dadurch gekennzeichnet, dass** die Polster (2, 3) mit einer Substanz beschichtet oder imprägniert sind, die einen Kühleffekt auf der Haut hat.

3. Vorrichtung gemäß Anspruch 1, **dadurch gekennzeichnet, dass** die Polster (2, 3) im wesentlichen aus einem hydrophilen Gelmaterial bestehen.

4. Vorrichtung gemäß der Ansprüche 1 bis 3, **dadurch gekennzeichnet, dass** ein Satz der Polster (3) seitwärts an einer Seite der elastischen Vorrichtung gegenüberliegt.

## Revendications

1. Dispositif pour un traitement d'acuponcture, plus spécifiquement pour stimuler les points d'acuponcture BL1 et BL2 des deux côtés de la racine du nez et au-dessus des yeux près de la racine du nez, **caractérisé en ce qu'**une attache élastique en forme d'arc (1) a des tampons (2, 3) agencés en paires symétriquement autour d'un plan de symétrie pour cette attache, lesquels tampons sont conçus pour exercer une pression contre lesdits points d'acuponcture lorsque l'arc est agencé au-dessus de la racine du nez de l'utilisateur.

2. Dispositif selon la revendication 1, **caractérisé en ce que** les tampons (2, 3) sont enduits ou imprégnés d'une substance qui possède un effet de refroidissement sur la peau.

3. Dispositif selon la revendication 1, **caractérisé en ce que** les tampons (2, 3) consistent essentiellement en une matière en gel hydrophile.

4. Dispositif selon les revendications 1 à 3, **caractérisé en ce qu'**un ensemble de tampons (3) fait face latéralement à un côté du dispositif élastique.
